# EUROPEAN PATENT APPLICATION

(11) **EP 4 131 273 A1**
(43) Date of publication of application: **08.02.2023**
(21) Application number: 21776181.6
(22) Date of filing: 23.03.2021
(51) Int. Cl.: G16B 25/00, G16B 20/00

(54) **METHOD FOR PROVIDING, ON BASIS OF HETEROLOGOUS ORGANISM-DERIVED GENETIC MARKER MATCHING, GENETIC TESTING SERVICE BY USING ONE OR MORE GENETIC MARKERS OF MODEL ORGANISM AND PATTERN INFORMATION THEREOF AS GENETIC MARKER INFORMATION OF TARGET ORGANISM**

(30) Priority: 26.03.2020 KR 20200036720
(71) Applicant: Clinomics Inc., Ulsan 44919 (KR)
(72) Inventor: BHAK, Jong Hwa, Ulju-gun Ulsan 44936 (KR); CHO, Yun Sung, Yongin-si Gyeonggi-do 16953 (KR)
(74) Representative: Frenkel, Matthias Alexander
(86) International application number: PCT/KR2021/003589
(87) International publication number: WO 2021/194227

(57) **Abstract**

Provided is a method for providing, on the basis of genetic marker matching of heterologous organisms, a genetic testing service by using genetic markers of a model organism as genetic markers of a target organism, the method comprising the steps of selecting at least one genetic marker of a pre-stored model organism; comparing genomic information of the model organism to genomic information of a target organism to be subjected to a genetic testing service, when the selected at least one genetic marker is a previously known genetic marker through pre-analyzed information; and providing a genetic mutation-based genetic report about a target organism on the basis of the results of comparing genomic information of target and model organisms.

## Description

### Technical Field

The present invention is a method for providing a genetic test service using one or more genetic markers of representative model organisms, a set of genetic markers, and distribution pattern information of the markers as genetic marker information of a target organism, in which the genetic markers are those of well-known genes and representative model organisms that have already been studied in depth scientifically and well annotated in the literature, and a method for finding out or predicting genetic variation information and related functional information using a genetic marker of a pre-analyzed model organism as genetic marker information of a target organism to be tested.

### Background Art

A genetic marker refers to a genetic type or characteristic that indicates the phenotype of living organisms, including humans. So far, many studies have been conducted comparing normal and disease samples in humans. Many studies have compared samples with and without having excellent economic and symbolic traits in the population of livestock and pets. Genetic markers representing these phenotypes exist on the genome of living things or in the exogenous genome (epigenome). However, the full-length genome is vast, so the study mainly focused on important genes known to be fundamentally related to traits. Currently, due to the rise of the next-generation sequencing (NGS) technique, decoding (sequence) information and genetic traits are being excavated as a large amount of data. As many of these NGS data are increasing, research in functional genomics is accelerating. Many NGS data are being generated to elucidate the functions of the genome and exogenous genome for not only humans but also economic animals such as pets and livestock, and plants, and functions for many genomic regions, including quantitative trait locus (QTL), have been revealed.

Currently, a method for performing a genetic test using a genetic marker has been researched and developed. In this regard, prior art Korean Patent Publication No. 2019-0019395 (published on February 27, 2019) and Korean Patent No. 10-1325736 (published on November 08, 2013), respectively, disclose a configuration of obtaining information about at least one single nucleotide polymorphism marker associated with at least one trait among the genetic information of the subject, determining the score of at least one single nucleotide polymorphic marker according to the effect of at least one single nucleotide polymorphic marker on at least one trait in reference to the expression type of the at least one single nucleotide polymorphism marker, determining the score of at least one single nucleotide polymorphic marker according to the effect of at least one single nucleotide polymorphic marker on at least one trait, and estimating the aptitude of the subject based on the determined score of at least one single nucleotide polymorphic marker and a configuration for extracting mutation data mapped to a gene by sequencing a gene sample, calculating a hazard score that quantifies disorders that occur in gene function due to mutation data, and searching a gene network for submodules in which genes having a hazard score equal to or greater than a predetermined threshold are aggregated.

However, numerous genetic markers associated with various diseases and phenotypes have been identified for genetic testing, mainly in humans. In other non-human animals with high homology, only genetic markers associated with some phenotypes have been identified. In addition, since the revealed genetic marker is also a genetic marker corresponding to a specific disease or phenotype, genetic testing using a genetic marker other than a specific disease or phenotype is impossible. Accordingly, there is a need to develop a method for matching or transcribing to target organisms of different species in consideration of the genetic markers, genetic marker sets, or distribution pattern information of the markers of the model organism for which the genetic markers have been identified, such as humans. The function change is derived from the matched genetic markers and predicting the change in the trait function derived from a matched genetic marker and using it as a genetic marker of a target organism.

### Disclosure

### Technical Problem

One embodiment of the present invention matches or transcribes information such as genes, exogenous genes, or combinations and distribution patterns of these types of markers of a model organism in which genetic markers are identified, such as humans, to a target organism, and changes in trait function derived from matched genetic markers are predicted bioinformatically. Thus, it is immediately used as a genetic marker of a target heterogeneous organism. Therefore, it is possible to easily translate and match genetic markers that have not been revealed through experimental research or verification in the target organism. Accordingly, the pre-established information of the model organism theoretically or inferentially is used to bioinformatically find or predict and use the genetic marker of the target organism, which is the species to be tested without additional expensive new research and development. Thus, it has the advantage of being able to generate and provide a gene test report for the predicted function of the target organism quickly and easily. However, since it is based on theoretical reasoning, some unintentional sacrifices in accuracy may occur.

In conclusion, the method, according to an embodiment of the present invention, is a method of providing a genetic test service for a different species based on the use of a genetic marker of a model organism as a genetic marker of a target organism using genetic marker matching information of a heterogeneous organism. However, the technical task to be achieved by the present embodiment is not limited to the technical task described above, and other technical tasks may exist. Not only genes but also exogenous information can be matched and utilized in other organisms using the same principle. Even though it is referred to only as a gene in the present specification, the contents may be equally or similarly applied to the case of an exogenous gene.

### Technical Solution

As a technical means for achieving the above-described technical problem, an embodiment of the present invention comprises steps of selecting at least one gene or exogenous marker from a pre-stored model organism, comparing when the selected at least one genetic marker is a pre-disclosed genetic marker based on pre-analyzed information, the genomic information of the model organism and the genome information of the target organism to be used for the genetic testing service, and providing a gene mutation-based gene report for the target organism based on the comparison result of the genome information of the target organism and the model organism. In selecting a marker to be used for the target organism, the genetic marker selected may be a 1:1 relationship resulting from comparison through the above-described matching process, etc., and maybe a relationship of a set of such markers. If there is a pattern such as distribution in the genetic markers, additional information such as distribution pattern within the genetic region may be used together.

### Advantageous Effects

According to any one of the above-mentioned means for solving the problems of the present invention, gene markers of a set of markers of a model organism in which genetic markers are identified, such as humans, are matched or transcribed to a target organism, and changes in trait function derived from matched genetic markers are predicted to use as a genetic marker of a target organism. Therefore, it is possible to easily translate and match genetic markers that have not been revealed in the target organism. Accordingly, the pre-established information of the model organism is used to find or predict and use the genetic marker of the target organism, which is the species to be tested without additional new research and development. As a final result, it is possible to quickly and easily generate and provide a genetic test report for a target organism.

### Description of Drawings

Fig. 1 is a view for explaining a system for providing a genetic test service using a genetic marker of a model organism based on a genetic marker matching of a heterogeneous organism as a genetic marker of a target organism, according to an embodiment of the present invention.
Fig. 2 is a block diagram illustrating a genetic test service providing server included in the system of FIG. 1.
Fig. 3 is a flowchart illustrating an example in which a genetic testing service using a genetic marker of a model organism based on genetic marker matching of a heterogeneous organism as a genetic marker of a target organism is performed, according to an embodiment of the present invention.
Fig. 4 is an operation flowchart for explaining the process of comparing genome information between heterogeneous organisms of Fig. 3.
Fig. 5 is a flowchart illustrating a method of providing a genetic test service using a genetic marker of a model organism based on a genetic marker matching of a heterogeneous organism as a genetic marker of a target organism, according to an embodiment of the present invention.

### Mode for Invention

Hereinafter, embodiments of the present invention are described in detail with reference to the accompanying drawings so that those of ordinary skill in the art can easily implement them. However, the present invention may be embodied in various forms and is not limited to the embodiments described herein. In order to clearly explain the present invention in the drawings, parts irrelevant to the description are excluded, and like reference numerals are assigned to like parts throughout the specification.

Throughout the specification, when a part is "connected" with another part, this includes not only the case of being "directly connected" but also the case of being "electrically connected" with another element interposed therebetween. Also, when a part "includes" a certain component, it means that other components may be further included, rather than excluding other components, unless otherwise stated. It is to be understood that it does not preclude the possibility of the presence or addition of one or more other features, numbers, steps, operations, components, parts, or combinations thereof. For example, in the case of matching genetic markers between heterogeneous organisms, in addition to simple 1:1 matching, additional information such as a set of markers, a distribution pattern of markers, and an occurrence frequency pattern in each organism may be utilized in the final matching process.

The terms related to a degree "about," "substantially," and the like used throughout the specification is used in a sense at or close to the numerical value when manufacturing and material tolerances inherent in the stated meaning are presented and used to prevent, by unconscionable infringers, unreasonable exploitation of the disclosure in which exact or absolute figures are described to help the understanding of the present invention. As used throughout the specification of the present invention, the term "step for" or "step for" related to a degree does not mean "step for."

In this specification, a "part" includes a unit realized by hardware, a unit realized by software, and a unit realized using both. In addition, one unit may be implemented using two or more hardware, and two or more units may be implemented with one hardware.

In this specification, some of the operations or functions described as being performed by the terminal, apparatus, or device may be performed instead of by a server connected to the terminal, apparatus, or device. Similarly, some of the operations or functions described as being performed by the server may also be performed in a terminal, apparatus, or device connected to the server.

In this specification, some of the operations or functions described as mapping or matching with the terminal may be interpreted as mapping or matching the terminal's unique number or personal identification information, which is the identification data of the terminal.

Further, it should be understood that the term "gene" used in this specification always refers to including an epigenetic.

Hereinafter, the present invention is described in detail with reference to the accompanying drawings.

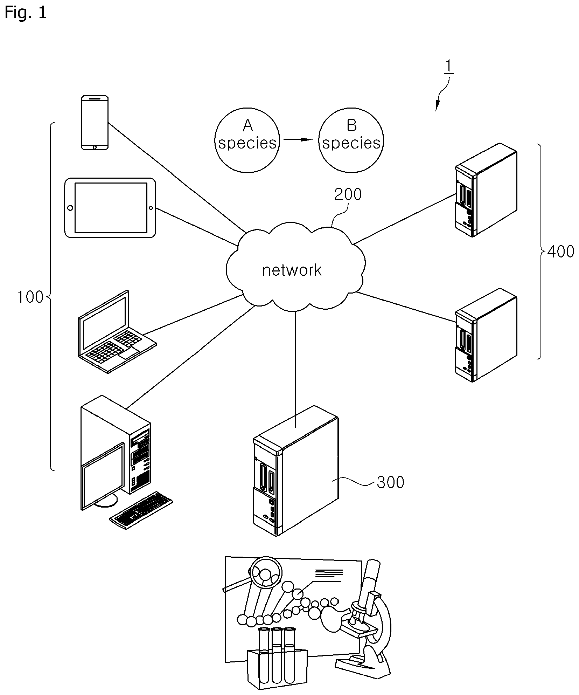

Fig. 1 is a view for explaining a system for providing a genetic test service using a genetic marker of a model organism based on a genetic marker matching of a heterogeneous organism as a genetic marker of a target organism, according to an embodiment of the present invention. Referring to Fig. 1, a system for providing genetic test service 1 using a genetic marker of a model organism based on genetic marker matching of a heterogeneous organism as a genetic marker of a target organism comprises at least one user terminal 100, a genetic test service providing server 300, at least one database server 400. However, the system for providing genetic test service 1 that uses the genetic marker of a model organism based on genetic marker matching of a heterogeneous organism of FIG. 1 as a genetic marker of a target organism is only an embodiment of the present invention. Accordingly, the present invention is not limitedly interpreted through Fig. 1.

In this case, each component of Fig. 1 is generally connected through a network 200. For example, as shown in Fig. 1, the at least one user terminal 100 may be connected to the genetic test service providing server 300 through the network 200. Further, the genetic test service providing server 300 may be connected to the at least one user terminal 100 and the at least one database server 400 through the network 200. Further, the at least one database server 400 may be connected to the genetic test service providing server 300 through the network 200.

In this case, the network refers to a connection structure in which information exchange is possible between each node, such as a plurality of terminals and servers. Such networks include RF, 3rd generation partnership project (3GPP) network, and Long Term (LTE). Evolution) network, 5th generation partnership project (5GPP) network, world interoperability for microwave access (WiMAX) network, Internet, local area network (LAN), wireless local area network (Wireless LAN), wide area network (WAN), personal area network (PAN), Bluetooth network, NFC network, satellite broadcasting network, an analog broadcasting network, digital multimedia broadcasting (DMB) network, and the like are included but are not limited thereto.

In the following, it is apparent that the term at least one is defined as a term including the singular and the plural, and although the term at least one does not exist, each element may exist in the singular or plural and may mean the singular or plural. Further, each component is provided in a singular or a plurality, which may be changed, according to an embodiment.

The at least one user terminal 100 may be a terminal of a user who requests a genetic test for themselves, others, or other biological species using a genetic test service-related web page, app page, program, or application that uses the genetic marker of a model organism based on genetic marker matching of a heterogeneous organism as a genetic marker of a target organism.

In this case, the at least one user terminal 100 may be a terminal that requests a genetic test result for a genetic sample of a subject or a test species to the genetic test service providing server 300. Further, the at least one user terminal 100 may be a terminal that receives a genetic test result for a subject or a test species from the genetic test service providing server 300 as a genetic test report.

Here, the at least one user terminal 100 may be implemented as a computer capable of accessing a remote server or terminal through a network. Here, the computer may include, for example, navigation, a laptop equipped with a web browser, a desktop, and a laptop. In this case, the at least one user terminal 100 may be implemented as a terminal capable of accessing a remote server or terminal through a network. The at least one user terminal 100 is, for example, a wireless communication device with guaranteed portability and mobility and may include all kinds of handheld-based wireless communication devices such as navigation, personal communication system (PCS), global system for mobile communications (GSM), personal digital cellular (PDC), personal handy-phone system (PHS), personal digital assistant (PDA), international mobile telecommunication (IMT)-2000, code division multiple access (CDMA)-2000, W-code division multiple access (W-CDMA), Wireless broadband internet (Wibro) terminal, a smartphone, a smart pad, a tablet PC, etc.

The genetic test service providing server 300 may be a server providing a genetic test service's web page, app page, program, or application using a genetic marker of a model organism based on genetic marker matching of a heterogeneous organism as a genetic marker of a target organism. Further, the genetic test service providing server 300 may be a server as follows: when the genetic test service providing server 300 receives a genetic test request for a subject or a test species from the user terminal 100, the genetic test service providing server 300 selects at least one genetic marker from the model organism. Further, when the selected genetic marker is a genetic marker that has already been identified and well known through research, etc., and is of high importance, the model organism's genome information and the target organism's genome information are compared. Further, when there is a genetic marker with a high matching rate or similarity, a functional change of the genetic marker of the target organism corresponding to a subject or species to be tested is predicted to generate a genetic test report on the genetic mutation of the target organism. At this time, the degree of publicity may be determined depending on whether the search is performed in the at least one database server 400 or whether the importance has been studied and revealed, or what the level of importance is even if it is discovered but various variables other than the listed parameters and conditions may exist.

Here, the genetic test service providing server 300 may be implemented as a computer capable of accessing a remote server or terminal through a network. Here, the computer may include, for example, navigation, a laptop equipped with a web browser, a desktop, and a laptop.

The at least one database server 400 may be a server as follows: the server 400 may use or not use the genetic test service-related web page, app page, program, or application that uses the genetic marker of the model organism based on genetic marker matching of a heterogeneous organism as a genetic marker of the target organism. Further, when the genetic test service providing server 300 requests a response to the existence of the genetic marker or whether it is important or well known, the at least one database server 400 transmits the response data to the genetic test service providing server 300. In this case, the at least one database server 400 may be a server that collects, maps, and stores information on at least one genetic marker, a reference standard genome map, importance, and whether or not it is well known.

When there is no importance, knowledge, or a reference standard genome map, the genetic test service providing server 300 builds the re-stored information for each genetic marker. Further, when the presence or absence of a genetic marker is received as a response (Ack) in the at least one database server 400, it may be implemented to integrate and use two pieces of information based on a genetic marker.

Here, the at least one database server 400 may be implemented as a computer that may connect to a remote server or terminal through a network. Here, the computer may include, for example, navigation, a laptop equipped with a web browser, a desktop, and a laptop. In this case, the at least one database server 400 may be implemented as a terminal capable of accessing a remote server or terminal through a network. The at least one database server 400 is, for example, a wireless communication device with guaranteed portability and mobility and may include all kinds of handheld-based wireless communication devices such as navigation, personal communication system (PCS), global system for mobile communications (GSM), personal digital cellular (PDC), personal handy-phone system (PHS), personal digital assistant (PDA), international mobile telecommunication (IMT)-2000, code division multiple access (CDMA)-2000, W-code division multiple access (W-CDMA), Wireless broadband internet (Wibro) terminal, a smartphone, a smart pad, a tablet PC, etc.

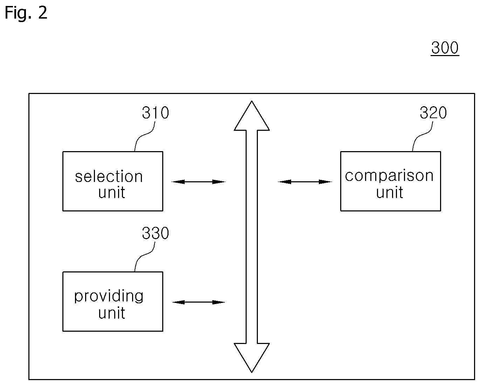

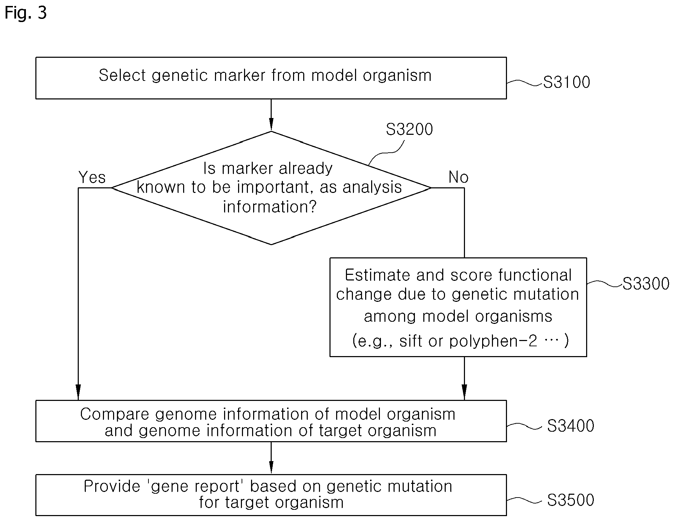

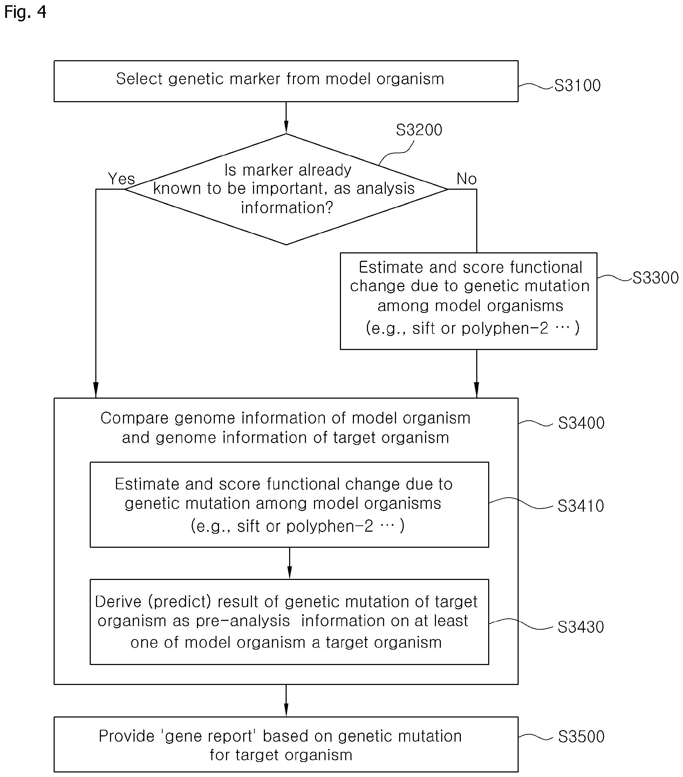

Fig. 2 is a block diagram illustrating a genetic test service providing server included in the system of FIG. 1, Fig. 3 is a flowchart illustrating an example in which a genetic testing service using a genetic marker of a model organism based on genetic marker matching of a heterogeneous organism as a genetic marker of a target organism is performed, according to an embodiment of the present invention. Fig. 4 is an operation flowchart for explaining the process of comparing genome information between heterogeneous organisms of Fig. 3.

Referring to Fig. 2, the genetic test service providing server 300 may include a selection unit 310, a comparison unit 320, and a providing unit 330.

When the gene test service providing server 300 according to an embodiment of the present invention, or another server (not shown) operating in conjunction with the same transmits an application, program, app page, web page of a genetic test service, and the like that uses the genetic marker of a model organism based on genetic marker matching of a heterogeneous organism as a genetic marker of a target organism to the at least one user terminal 100 and the at least one database server 400, the at least one user terminal 100 and the at least one database server 400 may install or open the application, program, app page, web page of a genetic test service and the like that uses the genetic marker of a model organism based on genetic marker matching of a heterogeneous organism as a genetic marker of a target organism. Further, the service program may be driven in the at least one user terminal 100 and the at least one database server 400 using a script executed in a web browser. Here, the web browser is a program that enables the use of a web (world wide web) service and refers to a program that receives and displays hypertext written in a hypertext markup language (HTML). Examples include Netscape, Explorer, and Chrome. Further, the application means an application on the terminal, for example, includes an app (app) executed in a mobile terminal (smartphone).

Referring to FIG. 2, the selection unit 310 may select at least one genetic marker from a pre-stored model organism. In this case, the model organism means an organism in which a genetic marker corresponding to a phenotypic gene indicating a disease or physical characteristic has been previously identified through many studies and experiments, such as humans or mice.

When the genetic marker A of the human species has been identified for disease B or physical characteristics C through research and experimentation, the significance and association between the genetic marker A and disease B or between the genetic marker A and physical characteristics C have already been revealed. Thus, correspondence between genetic markers-diseases such as A-B and genetic markers-physical features such as A-C may be known. When it is identified that the genetic marker of A is present in any patient Z, it can be predicted that the disease B and the physical characteristics C have appeared, are about to appear, or have the potential to appear. Based on this, one embodiment of the present invention reveals the matching rate of genetic markers of not only homogeneous organisms but also heterogeneous organisms. For example, humans and mice, humans and cats, humans and horses, and humans and dogs take advantage of the fact that when the gene marker A that appears in humans is also present in dogs, it can be predicted that disease B and physical characteristics C will appear. Of course, the above description is a simplified process, and a detailed description is given later. However, when the gene marker A is the same in humans and dogs, and it is predicted that a functional change corresponding to the genetic mutation of the gene marker A will occur, even in dogs, it can be predicted that a dog will also develop disease B or physical characteristics C.

To this end, when the selection unit 310 selects at least one genetic marker from the pre-stored model organism, the at least one genetic marker may be a genetic marker selected from a preset functional region of the pre-stored model organism. In this case, the preset functional region may be a region including any one or a combination of at least one of a protein-coding region, a 5', 3' region, a promoter region, and a splice region, but is not limited to the described above. It may also be a genomic region having a lot of functional information, such as an intergenetic region, an intron, or a combination thereof. Further, the preset functional region may be a region with a lot of functional significance among disease, physical characteristics, and phenotypic, genetic markers pre-studied for a model organism, such as an exogenously important region. Genetic markers may be selected from these preset functional regions. However, the preset functional region is not limited to a specific genomic region.

Further, when the selection unit 310 selects at least one genetic marker from the pre-stored model organism, the at least one genetic marker may include a gene mutation type corresponding to any one or a combination of at least one single nucleotide variant, copy number variation, indels, structural variation, epigenomic markers and protein expression among RNA expression. In this case, the at least one genetic marker may be any type of genetic mutation. The reason is that if the gene is mutated in the model organism, the gene can also be mutated in the target organism. Of course, it is not immediately applied because it is used after a pre-stored functional change prediction program, which will be described later, identifies functional changes due to genetic mutations. Further, at least one genetic marker type is not limited to a specific mutation type, is not limited to those listed, and is not excluded for reasons not listed.

The comparison unit 320 may compare the genome information of the target organism for the genetic testing service and the genome information of the model organism when the selected at least one genetic marker is a pre-published genetic marker based on pre-analyzed information. At this time, when the at least one selected genetic marker is not a pre-published genetic marker based on pre-analyzed information, the comparison unit 320 predicts a functional change due to genetic mutation through a pre-stored function change prediction program. If so, at least one genetic marker whose score corresponding to the predicted functional change exceeds a preset score may be selected. In this case, the pre-stored functional change prediction program may be a program for predicting the importance of a function, such as Sift or Polyphen-2, but the type of the program is not limited to those described above. This is because the functional change prediction program may be easily implemented in various ways, even if it is not the aforementioned program.

The comparison unit 320 may compare the reference standard genome map corresponding to the genome information of the model organism and the reference standard genome map corresponding to the genome information of the target organism by whole genome alignment. For example, when comparing the reference standard genome map of humans, which is a model organism, with the reference standard genome map of dog, which is the target organism, major genetic markers present on the genome map are matched (liftover) and the matching rate exceeds the preset value, it can be considered that the two genetic markers perform the same function. The reason is that the genome means all deoxyribonucleic acid (DNA), including genes in living things, and contains all the biological information necessary to make living things and sustain life.

Understanding the genome information of an organism plays an important role in understanding the organism's life phenomenon, the relationship between the genotype and phenotype genes, and the traits of the organism formed by the influence of the environment. For example, if a gene directs hepatocytes to remove excess cholesterol from the bloodstream, this gene instructs hepatocytes to make a specific protein, and the protein produced is responsible for removing excess cholesterol. However, if this gene is mutated or changed, the produced protein may not work properly or may not be made at all, making it impossible to remove excess cholesterol. It is important to find out the nucleotide sequence of the genome in order to find the location of the gene associated with such familial hypercholesterolemia and to detect the modification. As such, an embodiment of the present invention performs whole genome alignment on a reference standard genome map to match a model organism, a genetic marker and a set of markers. Particularly, a genetic marker preset to be important on the genome of a target organism similar or identical to the model organism, and when the matching result exceeds the preset value or aligned, it can be predicted that a phenotype such as a disease or physical characteristic of the model organism will be exhibited in the target organism in the order of the genetic marker with the highest matching rate.

Meanwhile, when the selected at least one genetic marker is a pre-published genetic marker based on pre-analyzed information, the comparison unit 320 compares the genome information of the target organism for genetic test service to the genome information of the model organism. Further, when the reference standard genome map corresponding to the genome information of the target organism does not exist, after aligning the gene sequence of the target organism to the reference standard genome map of the model organism (sequence alignments), at least selected After sequence-aligning the gene sequence of the target organism to the reference standard genome map of the model organism. Further, after matching (Liftover) with selected at least one genetic marker present on the genomic information of the model organism, The genome information of the target organism can be mapped to the genome. Assuming that a reference standard genome map of a target organism of, for example, a cat, does not exist, the cat's gene sequence is aligned to the human reference standard genome map. Further, when matching with the selected at least one genetic marker, and the matching rate exceeds a preset value or satisfies a preset rank when sorted in ascending order, it can be seen that a genetic marker in a region with a high matching rate is also present in cats, and it is possible to generate a genome map through this.

The providing unit 330 may provide a gene report based on a gene mutation for the target organism based on the comparison result of the genomic information of the target organism and the model organism. At this time, when as a result of the comparison, the matching rate of pre-selected at least one genetic marker on the genomic information of the model organism to the target organism exceeds a preset value, the providing unit 330 predicts functional change due to genetic mutation of at least one of the target organism exceeding the preset value through pre-stored function change prediction program. Further, the providing unit 330 sets at least one genetic marker for which the score corresponding to the predicted functional change exceeds a preset score as a genetic marker that is reliable in the functional association of the genetic mutation of the target organism and the model organism and the functional change of the genetic mutation, thereby creating a genetic report.

In this case, the level, i.e., the degree at which it is considered that the functional relevance and the reliability of the functional change of the gene mutation exist in both the target organism and the model organism, may be classified differently, according to the following four examples. That is, each reliability score may be given differently, which is described below.

### [First embodiment]

The comparison unit 330 provides the gene mutation-based gene report for the target organism based on the comparison result of the genomic information of the target organism and the model organism. When, as a comparison result, the matching rate of the pre-selected at least one genetic marker on the genomic information of the model organism to the target organism exceeds a preset value, and the at least one genetic marker of the target organism exceeding the preset value is a functionally well-known genetic mutation marker in the model organism, the genetic mutation of the model organism is applied to the target organism. The reliability of the functional change of the genetic mutation may be set at a lower level. Reliability may be classified into a low level, a median level, and a high level. However, in addition to the above-mentioned level, it may be given in points or percentages. The first embodiment may be given the lowest score, the second and third embodiments may be given a higher score than the first embodiment, and the fourth embodiment may be given the highest score. In the first embodiment, for example, a genetic mutation marker that is well known functionally in a human model organism is applied to the target organism as it is. In this case, it is assumed that the existing scientific research results have the same or similar effect on other species. In this case, it is assumed that the mutation information of a model organism in a disease-related database such as ClinVar or Omim, for example, a human genome genetic mutation is a meaningful mutation having the same function in the target species. Since all data is based on assumptions, the reliability score can give the low level the lowest.

### [Second embodiment]

The comparison unit 330 provides the gene mutation-based gene report for the target organism based on the comparison result of the genomic information of the target organism and the model organism. When, as a comparison result, i) the matching rate of the pre-selected at least one genetic marker on the genomic information of the model organism to the target organism exceeds a preset value, ii) at least one genetic marker of the model organism corresponding to the at least one genetic marker of the target organism exceeding a preset value is not a genetic mutation marker functionally well known in the model organism, and iii) the function change score of the pre-stored functional change prediction program of at least one genetic marker of the target organism exceeding a preset value exceeds the preset score, iv) the genetic mutation of the model organism may be applied to the target organism, and the reliability may be set at a median level. There is no information about the functional change in the second embodiment because there is no significant study on the model organism. However, when the genetic mutation on the genome map of the matched (liftover) target organism has a large functional change in the result of driving it with a function change prediction program such as Sift or Polyphen-2, it may be used as a mutation that predicts the genetic mutation and functional change of another target species, that is, the target organism. This may have a slightly higher score than the first embodiment described above. For example, the reliability may be given a median level. Further, the reliability may be calculated by a scoring method that gives more points than in the first embodiment, as described above.

### [Third embodiment]

The providing unit 330 provides the gene mutation-based gene report for the target organism based on the comparison result of the genomic information of the target organism and the model organism. When, as a comparison result, i) the matching rate of the pre-selected at least one genetic marker on the genomic information of the model organism to the target organism exceeds a preset value, ii) at least one genetic marker of the model organism corresponding to the at least one genetic marker of the target organism exceeding a preset value is a genetic mutation marker that is not included in a database pre-established in the model organism, and iii) the functional change score of the pre-stored functional change prediction program exceeds the preset score, iv) the genetic mutation of the model organism may be applied to the target organism, and the reliability may be set at a median level. In this case, although genetic mutations are not known in the mutation information database already known in the model organism, that is, Clinvar, which is a pre-established database, i) if a prediction is made by a functional change prediction program such as Sift or Polyphen-2, it means that the functional change is expected to appear. In this case, as described above, the score may be slightly higher than in the first embodiment, and for example, the reliability may be given a median level. Further, the reliability may be calculated by a scoring method that gives more points than in the first embodiment, as described above. Here, the scores of the second and third embodiment may be the same, but different settings may be made. Further, it ispossible to set the level differently by subdividing the lower level among the median level.

Further, there is one more case in the third embodiment. The providing unit 330 provides a gene mutation-based gene report for the target organism based on the comparison result of the genome information of the target organism and the model organism. When, as a result of the comparison, i) the matching rate of the pre-selected at least one genetic marker on the genomic information of the model organism to the target organism exceeds a preset value, ii) at least one genetic marker of the model organism corresponding to the at least one genetic marker of the target organism exceeding a preset value is a genetic mutation marker that is not included in a database pre-established in the model organism, and iii) the functional change score of the pre-stored functional change prediction program of at least one genetic marker of the target organism exceeding a preset value exceeds the preset score, iv) the genetic mutation of the model organism may be applied to the target organism, and the reliability may be set at a median level. In this case, although genetic mutations are not known in the mutation information database already known in the model organism, that is, Clinvar, which is a pre-established database, if a prediction is made by a functional change prediction program such as Sift or Polyphen-2, this refers to a case in which a high score is obtained from a functional change prediction program such as Sift or Polyphen-2 even in the gene sequence on the genome map of the target organism that matches this.

### [Fourth embodiment]

The comparison unit 330 provides the gene mutation-based gene report for the target organism based on the comparison result of the genomic information of the target organism and the model organism. When, as a comparison result, i) the matching rate of the pre-selected at least one genetic marker on the genomic information of the model organism to the target organism exceeds a preset value, ii) the at least one genetic marker of the target organism exceeding the preset value is a functionally well-known genetic mutation marker in the model organism, and iii) the functional change score of the pre-stored functional change prediction program exceeds the preset score, iv) the genetic mutation of the model organism may be applied to the target organism, and the reliability may be set at a higher level. In this case, it is already known in the model organism that an important functional change occurs when a genetic mutation occurs. Further, when a functional change prediction program such as Sift and Polyphen-2 is run on the genetic mutation on the genome map of matched (liftover) different species, that is, the target organism, if the functional change continues to be large, it is to be used as a genetic mutation to predict the genetic mutation and functional change of the target organism, and it is to set the reliability as high as the intensity of the functional change is large. In this case, as described above, the score may be higher than that of the second or third embodiment; for example, the reliability may be given a higher level.

Further, the reliability may be calculated by a scoring method in which more points are given than in the second or third embodiment as described above. Overall, the first embodiment has the lowest score, the second embodiment and the third embodiment are higher than the first embodiment, the second embodiment and the third embodiment have the same score or are within the error range, and the fourth embodiment may be given a higher score than the second embodiment or third embodiment. Reliability can be summarized as follows: Reliability of embodiment 1 < Reliability of embodiment 2 ≒ Reliability of embodiment 3 < Reliability of embodiment 4.

Hereinafter, an operation process, according to the configuration of the genetic test service providing server of Fig. 2, is described in detail with reference to Figs. 3 and 4 as an example. However, it is apparent that the embodiment is only one of various embodiments of the present invention, and it is not limited thereto.

Referring to Fig. 3, the genetic test service providing server 300 selects a genetic marker from the model organism (S3100) and confirms that the selected genetic marker is classified as important based on pre-analyzed information and is a known genetic marker (S3200) as a result of confirmation if the information is i) pre-analyzed, ii) classified as important, and iii) is a known genetic marker, the genome information of the model organism and the genome information of the target organism are compared. If any one of i) to iii) or at least one combination thereof is not satisfied, the genetic test service providing server 300 predicts functional change due to genetic mutation in the model organism and performs scoring. Those with a high score may be utilized by making assumptions and predictions that there will be important functional changes even in the target organism, which is the target species.The score is not limited to any numerical value because the set value may differ for each genetic marker or mutation.

Next, the genetic test service providing server 300 compares the model organism's genome information with the target organism's genome information. At this time, referring to Fig. 4, scoring is performed by predicting due to genetic mutation in the target organism (S3410), and the genetic mutation result of the target organism is predicted based on the analyzed information of the model organism and the target organism, and reliability may be given as described above. The analyzed information may be research result information but is not limited thereto. Different scores may be given to reliability, as described above. Then, finally, the genetic test service providing server 300 may provide a gene mutation-based gene report for the target organism.

As described above, matters not described for the method of providing a genetic test service using the genetic marker of a model organism based on genetic marker matching of a heterogeneous organism as a genetic marker of a target organism of Figs. 2, 3, and 4 are the same or may be easily inferred from the description of the method of providing a genetic test service using the genetic marker of a model organism based on genetic marker matching of a heterogeneous organism as a genetic marker of a target organism of Fig 1. Thus, the description below is excluded.

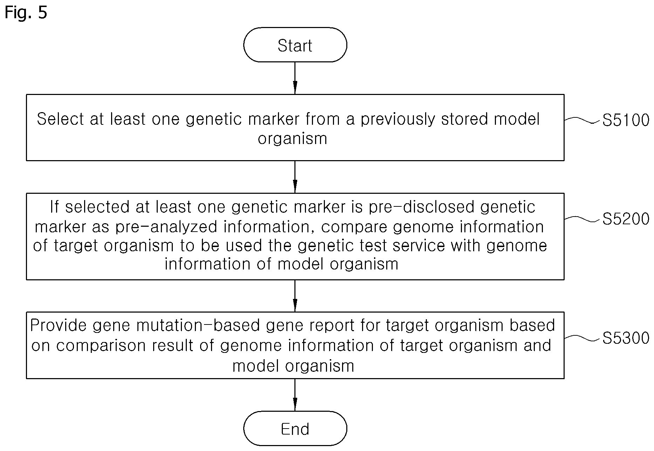

Fig. 5 is a view showing a process of transmitting and receiving data between respective components included in a system for providing genetic test service using a genetic marker of a model organism based on genetic marker matching of a heterogeneous organism of Fig. 1,according to an embodiment of the present invention. Hereinafter, an example of a process in which data is transmitted and received between the respective components is described with reference to Fig. 5. However, it is apparent to those skilled in the art that the present application is not limited to such an embodiment. According to the various embodiments described above, the data transmission/reception process shown in Fig. 5 may be changed.

Referring to Fig. 5, the genetic test service providing server selects at least one genetic marker from a pre-stored model organism (S5100). It compares when the selected at least one genetic marker is a pre-published genetic marker as pre-analyzed information, the genome information of the target organism, and the genome information of the model organism to be used for the genetic testing service (S5200), and provides a gene mutation-based gene report for the target organism based on the comparison result of the genome information of the target organism and the model organism (S5300).

The order between the above-described steps S5100 to S5300 is merely an example, and the present invention is not limited thereto. That is, the order between the above-described steps S5100 to S5300 may be mutually changed, and some of these steps may be simultaneously executed or deleted.

As described above, matters not described for the method of providing a genetic test service using the genetic marker of a model organism based on genetic marker matching of a heterogeneous organism as a genetic marker of a target organism of Fig. 5 are the same or may be easily inferred from the description of the method of providing a genetic test service using the genetic marker of a model organism based on genetic marker matching of a heterogeneous organism as a genetic marker of a target organism of Figs 1, 2, 3, and 4. Thus, the description below is excluded.

A method for providing a genetic test service using a genetic marker of a model organism based on genetic marker matching of a heterogeneous organism as a genetic marker of a target organism, according to an embodiment described with reference to Fig. 5, may also be implemented in the form of a recording medium containing instructions executable by a computer, such as an application or program module executed by a computer. Computer-readable media may be any available media accessed by a computer and includes volatile and nonvolatile media, removable and non-removable media. Further, computer-readable media may include all computer storage media. Computer storage media includes both volatile and nonvolatile, removable, and non-removable media implemented in any method or technology for storing information, such as computer-readable instructions, data structures, program modules, or other data.

The method of providing a genetic test service using a genetic marker of a model organism based on genetic marker matching of a heterogeneous organism as a genetic marker of a target organism, according to an embodiment of the present invention as described above may be executed by an application basically installed in a terminal (This may include programs included in the platform or operating system installed by default in the terminal). It may be executed by an application (i.e., a program) directly installed in the master terminal by a user through an application-providing server such as an application store server, an application, or a web server related to the corresponding service. In this sense, the method for providing a genetic test service using a genetic marker of a model organism based on genetic marker matching of a heterogeneous organism as a genetic marker of a target organism, according to an embodiment of the present invention, as described above may be implemented by an application (i.e., a program) basically installed in a terminal or directly installed by a user and may be recorded in a computer-readable recording medium such as a terminal.

The above description of the present invention is for illustration, and those of ordinary skill in the art to which the present invention pertains can understand that it can be easily modified into other specific forms without changing the technical spirit or essential features of the present invention. Therefore, it should be understood that the embodiments described above are illustrative in all respects and not restrictive. For example, each component described as a single type may be implemented in a distributed manner, and likewise, components described as distributed may also be implemented in a combined form.

The scope of the present invention is indicated by the following claims rather than the above-detailed description, and all changes or modifications derived from the meaning and scope of the claims and their equivalent concepts should be interpreted as being included in the scope of the present invention.

## Claims

1. A method of providing a genetic test service using a genetic marker of a model organism based on a genetic marker matching of a heterogeneous organism as a genetic marker of a target organism, the method comprising steps of:
selecting at least one genetic marker from a pre-stored model organism;
comparing, when the selected at least one genetic marker is a pre-published genetic marker as pre-analyzed information, the genome information of the target organism of the genetic testing service with the genome information of the model organism; and
generating and providing a gene mutation-based gene report for the target organism based on a comparison result of genomic information between the target organism and the model organism.

2. The method of claim 1, wherein in step of selecting at least one genetic marker from the pre-stored model organism, the at least one genetic marker is a genetic marker selected from a predetermined functional region of the pre-stored model organism, and
wherein the predetermined functional region is a region comprising at least one of a protein coding region, a 5' and 3' region, a promoter region, and a splice region or any one combination thereof.

3. The method of claim 1, wherein in step of selecting at least one genetic marker from the pre-stored model organism, the at least one genetic marker comprises a gene mutation type corresponding to any one of single nucleotide variants, copy number variations, indels, structural variations, epigenomic markers, protein gene expression among RNA gene expression or at least one combination thereof.

4. The method of claim 1, wherein step of comparing the genome information of the target organism of the genetic testing service with the genome information of the model organism comprises step of:
comparing, when the selected at least one genetic marker is a pre-published genetic marker as pre-analyzed information, the genome information of the target organism of the genetic testing service with the genome information of the model organism; or
predicting, when the selected at least one genetic marker is not a pre-published genetic marker as pre-analyzed information, functional change due to genetic mutation through a pre-stored function change prediction program and selecting at least one genetic marker whose score corresponding to the predicted functional change exceeds a preset score.

5. The method of claim 1, wherein step of comparing, when the selected at least one genetic marker is a pre-published genetic marker as pre-analyzed information, the genome information of the target organism of the genetic testing service with the genome information of the model organism comprises step of:
performing comparison by performing whole genome alignment between the reference standard genome map corresponding to the genome information of the model organism and the reference standard genome map corresponding to the genome information of the target organism.

6. The method of claim 1, wherein step of comparing, when the selected at least one genetic marker is a pre-published genetic marker as pre-analyzed information, the genome information of the target organism of the genetic testing service with the genome information of the model organism comprises step of:
sequence-aligning, when the reference standard genome map corresponding to the genome information of the target organism does not exist, the gene sequence of the target organism to the reference standard genome map of the model organism, then performing liftover by genome-wide matching with the selected at least one genetic marker present on the genomic information of the model organism, and then genomically mapping the genome information of the target organism.

7. The method of claim 1, wherein step of generating and providing a gene mutation-based gene report for the target organism based on a comparison result of genomic information between the target organism and the model organism comprises step of:
predicting, when as a result of the comparison, the matching rate of the selected at least one genetic marker on the genomic information of the model organism to the target organism exceeds a preset value, functional change due to genetic mutation through a pre-stored function change prediction program on the at least one genetic marker of the target organism that exceeds the preset value;
setting at least one genetic marker whose score corresponding to the predicted functional change exceeds the preset score as a genetic marker that is reliable in the functional association of the genetic mutation between the target organism and the model organism and the functional change of the genetic mutation to generate the gene report.

8. The method of claim 1, wherein step of generating and providing a gene mutation-based gene report for the target organism based on a comparison result of genomic information between the target organism and the model organism comprises step of:
applying, when as a result of the comparison, the matching rate of the selected at least one genetic marker on the genomic information of the model organism to the target organism exceeds a preset value, and when the at least one genetic marker of the target organism that exceeds the preset value is a genetic mutation marker that is functionally well known in the model organism, the genetic mutation of the model organism to the target organism and setting the functional change reliability of the genetic mutation to a lower level, and
wherein the reliability is classified into a low level, a median level, and a high level.

9. The method of claim 1, wherein step of generating and providing a gene mutation-based gene report for the target organism based on a comparison result of genomic information between the target organism and the model organism comprises step of:
applying, when as a result of the comparison, the matching rate of the selected at least one genetic marker on the genomic information of the model organism to the target organism exceeds a preset value, and when at least one genetic marker of the model organism corresponding to the at least one genetic marker of the target organism that exceeds the preset value is not a genetic mutation marker that is functionally well known in the model organism, and the functional change score of the pre-stored functional change prediction program on the at least one genetic marker of the target organism that exceeds the preset value, exceeds the preset score, the genetic mutation of the model organism to the target organism and setting the reliability to a median level, and
wherein the reliability is classified into a low level, a median level, and a high level.

10. The method of claim 1, wherein step of generating and providing a gene mutation-based gene report for the target organism based on a comparison result of genomic information between the target organism and the model organism comprises step of:
applying, when as a result of the comparison, the matching rate of the selected at least one genetic marker on the genomic information of the model organism to the target organism exceeds a preset value, and when at least one genetic marker of the model organism corresponding to the at least one genetic marker of the target organism that exceeds the preset value is a genetic mutation marker not included in the database pre-built in the model organism, and the functional change score of the functional change prediction program pre-stored exceeds the preset score, the genetic mutation of the model organism to the target organism and setting the reliability to a median level, and
wherein the reliability is classified into a low level, a median level, and a high level.

11. The method of claim 1, wherein step of generating and providing a gene mutation-based gene report for the target organism based on a comparison result of genomic information between the target organism and the model organism comprises step of:
applying, when as a result of the comparison, the matching rate of the selected at least one genetic marker on the genomic information of the model organism to the target organism exceeds a preset value, and when at least one genetic marker of the model organism corresponding to the at least one genetic marker of the target organism that exceeds the preset value is a genetic mutation marker not included in the database pre-built in the model organism, and the functional change score of the pre-stored function change prediction program on the at least one genetic marker of the target organism that exceeds the preset value, exceeds the preset score, the genetic mutation of the model organism to the target organism and setting the reliability to a median level, and
wherein the reliability is classified into a low level, a median level, and a high level.

12. The method of claim 1, wherein step of generating and providing a gene mutation-based gene report for the target organism based on a comparison result of genomic information between the target organism and the model organism comprises step of:
applying, when as a result of the comparison, the matching rate of the selected at least one genetic marker on the genomic information of the model organism to the target organism exceeds a preset value, and when the at least one genetic marker of the target organism that exceeds the preset value is a genetic mutation marker functionally well known in the model organism, and the function change score of the pre-stored function change prediction program thereof exceeds the preset score, the genetic mutation of the model organism to the target organism and setting the reliability to a high level, and
wherein the reliability is classified into a low level, a median level, and a high level.

13. A computer-readable recording medium recording a program for executing the method of any one of claims 1 to 12.
